# EUROPEAN PATENT APPLICATION

(11) **EP 4 349 859 A1**
(43) Date of publication of application: **10.04.2024**
(21) Application number: 23153815.8
(22) Date of filing: 27.01.2023
(51) Int. Cl.: C07K 16/06, C07K 16/28

(54) **METHOD TO PURIFY ANTI-D IMMUNOGLOBULIN G FROM PLASMA**

(71) Applicant: CSL Behring AG, 3014 Bern (CH)
(72) Inventor: ALDER, Adrian, 3014 Bern (CH); SPIESS, Matthias, 3014 Bern (CH)
(74) Representative: Carpmaels & Ransford LLP

(57) **Abstract**

The invention provides a process for reducing protease activity in a solution comprising anti-D immunoglobulin G comprising the steps of i) providing a solution comprising anti-D immunoglobulin G; ii) contacting said solution with aluminium hydroxide gel at a pH of 4.70 to 4.90; and iii) separating the solution from the solid components. The use of these conditions reduces protease activity, preferably without reducing yield of anti-D immunoglobulin G.

## Description

### TECHNICAL FIELD

This invention is in the field of purification of therapeutics for pharmaceutical use from blood.

### BACKGROUND ART

### anti-D

Morbus haemolyticus neonatorum is the general designation for the haemolytic anaemia of fetuses and newborn babies caused by antibodies of the mother. Of greatest clinical significance for rhesus incompatibility is the rhesus antigen D (RhD; Rho) which can be found in approximately 85% of Caucasians in Europe. Individuals having the D-antigen are called Rh-positive. Individuals lacking the D-antigen are called Rh-negative. Antibodies of the specificity anti-D are the most common irregular rhesus antibodies and may arise during rhesus incompatible pregnancies (e.g. when a mother is Rh negative, and the child is Rh positive). Sensitisation occurs when the mother is exposed to the Rh positive blood for the first time (e.g. as a result of small numbers of foetal blood cells crossing into the mother's blood, exposure of the mother to her baby's blood during delivery, an invasive procedure during pregnancy such as amniocentesis, or chorionic villus sampling (CVS), injury to the mother's abdomen or as a result of a previous miscarriage or ectopic pregnancy). During this sensitisation process antibodies are generated to the Rhesus positive blood cells. In most cases the antibodies aren't generated sufficiently rapidly to harm a baby during a mother's first pregnancy but any subsequent Rh-positive babies would be at risk from the antibodies. In such subsequent pregnancies rapidly generated anti-Rhesus antibodies can cross the placenta and cause erythroblastosis fetalis. Sensitisation may also arise following transfusion of rhesus-incompatible blood.

Prophylaxis of rhesus sensitisation with immunoglobulin anti-D is of decisive importance for Rh-negative women of child-bearing age. Without treatment, up to 17% of the primigravidae with the rhesus constellation (mother Rh-negative, child Rh-positive) would become sensitised to the rhesus antigen in the course of pregnancy or during delivery. Anti-D preparations have been used successfully for over 30 years to prevent the rhesus sensitization of Rh-negative women to the rhesus factor D and thus to prevent anti-D related diseases among newborns, namely rhesus-erythroblastose in all its forms. The anti-D immunoglobulin acts by neutralising any RhD-positive antigens that may have entered the mother's blood during pregnancy to prevent the generation of antibodies. Anti-D immunoglobulin is generally administered to RhD-negative mothers during pregnancy, as a matter of routine, and/or if the mother experiences any bleeding, has an invasive procedure (such as amniocentesis), or in cases of abdominal injury.

In addition, anti-D is also used after mistransfusions of Rh-positive blood to Rh-negative recipients; the dosage is to be adapted thereby to the amount of inflow of Rh-positive erythrocytes.

Anti-D immunoglobulin is also used in the treatment of idiopathic thrombocytopenic purpura (ITP), specifically for RhD-positive children with chronic or acute ITP, adults with chronic ITP, and children and adults with ITP secondary to HIV infection. Anti-D is recommended as a first-line therapy for ITP, along with corticosteroids and intravenous immune globulin (IVIG).

### Existing processes for making anti D

Various processes for making anti-D immunoglobulin G are known. With certain existing processes (as set out e.g. in [1, 2]) anti-D immunoglobulin G is obtained using a chromatographic fractionation method including at least one cation-exchange column chromatography step, an anion-exchange step, and an aluminium hydroxide gel treatment.

In more detail, the immunoglobulin can be obtained from human plasma. The starting material is the plasma from Rh-negative donors sensitized to the rhesus factor D. The individual donation obtained through plasmapheresis is generally frozen and thawed at 0-4° C before fractionation, and pooled. The plasma fraction used for the cation exchange chromatography is purified using centrifugation to remove the cryoglobulins.

Prior to the cation exchange chromatography, the cryoglobulin-free plasma is treated to inactivate enveloped viruses. This treatment is carried out with 1% of the biocompatible, organic solvent tri(n-butyl) phosphate and a detergent (1% Triton X-100). The mixture of plasma, solvent, and detergent is then incubated at 30°C. A phase separation then takes place. The clear lower phase is subjected to cation exchange chromatography.

In this cation exchange step, the anti-D IgG from the pre-treated product is bound to a weak cation exchanger, with carboxymethyl (CM) as a functional group. This step removes most of the non IgG proteins; the solvent and detergents used for virus inactivation are likewise removed here. The anti-D IgG concentrated by this process step is then eluted from the ion exchange gel.

This anti-D IgG fraction is then purified further by treatment with a second, weak anion exchanger with diethylaminoethyl (DEAE) as the functional group, anti-D IgG not being bound to the ion exchangers under the selected conditions.

To reduce the concentration of further undesired components, such as proteases, the immunoglobulin solution is treated additionally with aluminium hydroxide gel (e.g. Alhydrogel).

To increase concentration, the purified anti-D IgG fraction is preferably bound a second time to a CM ion exchange gel and is eluted as concentrated as possible under suitable conditions. Following nanofiltration for virus removal (e.g. with Planova 15N nanofilters) the eluate is manufactured into the final product.

### Solvent detergent step

The existing methods for preparing anti-D from blood contain various viral removal steps since blood preparations may be contaminated with viruses, including pathogenic viruses that are deleterious to health. It is important to eliminate any contaminating viral activity if the preparation is to be administered to an individual. There are currently many different methods for inactivating or removing pathogenic viruses, including, e.g. filtration, wet or dry heat-inactivation, solvent/detergent (S/D) inactivation, pH inactivation, chemical inactivation, and/or ultraviolet/gamma irradiation inactivation. Of these, S/D inactivation is perhaps the most widely used virucidal method because nearly all the significant human pathogens are enveloped viruses susceptible to membrane disruption by solvents and detergents. In the S/D inactivation method, an organic solvent and a detergent are mixed with a fluid including the polypeptide being purified and incubated. The solvent creates an environment promoting aggregation between the detergent and the lipid membrane encapsulating the virus, and the detergent disrupts the interactions between molecules in this lipid membrane. Once disrupted, an enveloped virus can no longer bind to and infect a cell and is unable to reproduce because an intact lipid membrane is essential for such activities. Typical conditions used in accordance with the World Health Organization (WHO) guidelines are 0.3% tri(n-butyl) phosphate (TNBP) and 1 % Polysorbate 80 (PS 80, also known as polyoxyethylene (80) sorbitan monooleate or TWEEN^{®} 80) incubated at 24°C for a minimum of 6 hours, or 0.3% TNBP and 1 % polyoxyethylene octyl phenyl ether (TRITON^{®} X-100 (T100)) incubated at 24°C for a minimum of 4 hours (see ref. 3).

Although S/D treatment has become a standard technique, conventionally-used detergents such as T100 are now known to pose a serious environmental threat and now must be discontinued. When replacing these conventionally used detergents with other, more environmentally friendly detergents, any effects of the detergent substitution need to be understood and mitigated.

An existing preparation method for the production of anti-D was therefore modified in order to avoid the use of T100. When T100 was replaced with Polysorbate-20 (PS-20) it was initially observed that the level of activity of serine proteases (e.g. kallikrein-like activity) in certain intermediary stages increased, e.g. as assessed using chromogenic assays. Human plasma contains many components, and strict upper limits on the amount of various of these components in the final anti-D immunoglobulin G product are set by the regulatory authorities to ensure patient safety. It can be challenging to ensure effective removal of such contaminating proteins, without reducing the yield of the desired product. A challenge when devising purification strategies is therefore to obtain an appropriate balance between removal of contaminating proteins e.g. from the starting human plasma, with obtaining a sufficiently high yield of the desired product.

Amongst the components for which there are strict upper limits are prekallikrein activator (PKA), and kallikrein-like activity. It was, surprisingly, found by the inventors that by performing the downstream aluminium hydroxide gel step under certain conditions, serine protease activity (e.g. kallikrein-like activity) could be lowered effectively, and without reducing the yield of the anti-D immunoglobulin G. This is advantageous in general terms, but is particularly advantageous in the context of a method in which PS-20 has been used for the viral inactivation step, since the use of this detergent has been observed to slightly increase the levels of protease activity after this step. The result of the inventors' work is the ability to greatly reduce protease activity in an anti-D immunoglobulin G containing solution, without significantly reducing the yield of the anti-D immunoglobulin G. Indeed, it was observed that the protease activity that resulted from carrying out the aluminium hydroxide gel step in this way was lower than in the T100 based manufacturing method. The inventors have therefore identified conditions for an aluminium hydroxide gel adsorption step that is carried out on a solution comprising anti-D immunoglobulin G that reduces protease activity without significantly adversely affecting the yield of anti-D immunoglobulin.

### DISCLOSURE OF THE INVENTION

The invention is based on the inventors' surprising observation that performing a step of contacting a solution comprising anti-D immunoglobulin G with aluminium hydroxide gel at a pH of around 4.70 to 4.90 reduces protease activity in the resulting solution, but does so without significantly reducing the yield of anti-D immunoglobulin in the resulting solution. The inventors have identified these conditions as particularly effective in achieving this balance between retaining the product of interest (the anti-D immunoglobulin) whilst reducing the protease activity, which is undesirable in a product that is to be administered to patients.

Accordingly, the invention provides a process for reducing protease activity in a solution comprising anti-D immunoglobulin G comprising the steps of: i) providing a solution comprising anti-D immunoglobulin G; ii) contacting said solution with aluminium hydroxide gel at a pH of 4.70 to 4.90; and iii) separating the solution from the solid components.

The aluminium hydroxide gel is typically added in an amount of 0.10-0.20g/g protein, preferably about 0.13g/g protein. Step (ii) is typically carried out in a solution with a conductivity of 3.0 to 3.4 mS/cm, preferably in a solution which has a conductivity of about 3.2 mS/cm. This step is typically carried out by contacting the mixture generated in (ii) with the aluminium hydroxide gel for a period of 15 minutes to 2 hours.

The method typically results in a solution which has reduced protease activity and at least the same amount of anti-D or an increased amount of anti-D compared to the same process in which the pH in step (ii) is pH 5.5, e.g. directly after the aluminium hydroxide gel step and/or in the final product.

The process may comprise additional steps, such as an anion exchange step, which is carried out before the step of contacting said solution with aluminium hydroxide gel, which is typically a DEAE step. A cation exchange step may be carried out before the step of contacting said solution with aluminium hydroxide gel.

In certain embodiments, a solvent detergent virus inactivation step is carried out before the step of contacting said solution with aluminium hydroxide gel, optionally wherein said solvent detergent virus inactivation step is carried out using Tri (n-butyl) phosphate (TnBP) and Polysorbate-20 (PS-20). Preferred amounts of solvent and detergent are 1% TnBP (w/w) and 1.75% PS-20 (w/w).

Further additional steps including a cation exchange chromatography step after step (iii) and a virus filtration step after step (iii) may also be carried out and the further steps of one or more of (i) formulating the solution, optionally with glycine and/or albumin, and (ii) filling syringes with the solution may also be carried out.

In certain embodiments, the solution comprising anti-D immunoglobulin G is plasma from human Rh-negative donors who have been sensitised to rhesus factor D, or a fraction thereof, a solvent detergent virus inactivation step using TnBP (e.g. 1% (w/w)) and PS-20 (e.g. 1.75% (w/w)) is carried out before the step of contacting said solution with aluminium hydroxide gel and step (ii) is carried out by contacting said solution with aluminium hydroxide gel in an amount of 0.10-0.20g/g protein at a pH of 4.70 to 4.90 and a conductivity of 3.0 to 3.4 mS/cm.

In an exemplary embodiment the process comprises the steps of: a) providing plasma from human Rh-negative donors who have been sensitised to rhesus factor D or a fraction thereof, comprising anti-D immunoglobulin G; b) carrying out a solvent detergent virus inactivation step on the plasma or fraction thereof using Tri (n-butyl) phosphate (TnBP) and Polysorbate-20 (PS-20); c) carrying out a cation exchange chromatography step on the solvent detergent treated plasma or fraction thereof; d) carrying out an anion exchange step on the eluate from step c); d) contacting said solution with aluminium hydroxide gel in an amount of 0.10-0.20 g/g protein at a pH of 4.70 to 4.90 and a conductivity of 3.0 to 3.4 mS/cm; and separating the solution from the solid components; e) carrying out a further step of cation exchange chromatography on the solution from d); f) filtering the solution from e) to reduce the amount of any virus present; and g) one or more of (i) formulating the solution, optionally with glycine and/or albumin, and (ii) filling syringes with the solution.

The process including steps a) to d), a) to e), a) to f) and a) to g) of the above paragraph may provide a solution comprising a level of serine protease activity of less than 50 nkat/1, optionally less than 40, 30, 20, 15 nkat/1 and/or a kallikrein-like activity that is less than 40 ng/ml, optionally less than 30, 30, 20, 15 ng/ml. The process including steps a) to d) of the above paragraph may provide a solution comprising 0.2-0.5g/L IgG and/or 10-50µg/ml anti-D immunoglobulin G. The process including steps a) to e) of the above paragraph may provide a solution comprising 0.5-20g/L IgG and/or 400-1500µg/ml anti-D immunoglobulin G. The process including steps a) to f) of the above paragraph may provide a solution comprising 1-5g/L (e.g. 1.5-5g/L, 2-4g/L) immunoglobulin G and/or 50-200µg/ml (e.g. 75-175µg/ml, 100-150µg/ml) anti-D immunoglobulin G.

The invention further comprises a method of producing an anti-D immunoglobulin G-preparation from plasma from rhesus negative blood of rhesus factor D sensitized donors, or a plasma fraction thereof. The steps of the method are as set out above.

Accordingly, the invention provides, in a process for producing an anti-D immunoglobulin G-preparation from a solution comprising anti-D immunoglobulin G (e.g. plasma from rhesus negative blood of rhesus factor D sensitized donors or a plasma fraction thereof), the improvement consisting of contacting the solution comprising anti-D immunoglobulin G with aluminium hydroxide gel at a pH of 4.70 to 4.90 and separating the solution from the solid components. The pH range specified may result in reduced protease in the resulting solution without reducing the yield of the anti-D immunoglobulin product.

The invention also provides the use of aluminium hydroxide gel in reducing protease activity in a solution comprising anti-D immunoglobulin G (e.g. plasma from rhesus negative blood of rhesus factor D sensibilized donors or a plasma fraction thereof), wherein the solution is contacted with aluminium hydroxide gel at a pH of 4.70 to 4.90 and the solution is separated from the solid components.

### Solution comprising anti-D immunoglobulin G

The process of the invention may be a process for reducing protease activity in a solution comprising anti-D immunoglobulin G and/or a process for producing an anti-D immunoglobulin G-preparation. In either case the solution comprising anti-D immunoglobulin G is preferably plasma from rhesus negative blood of rhesus factor D sensitized donors, or a plasma fraction thereof.

The solution comprising anti-D immunoglobulin G may be obtained from donors. It may be obtained by plasmapheresis, in which whole blood is removed from the donor, blood cells and plasma are separated, and the blood cells are returned while the plasma is collected, or by collecting whole blood and separating blood cells and plasma without returning other blood components to the body.

The process of separating blood components is generally referred to as fractionation and blood or plasma fractions result from various techniques to separate blood components (plasma is itself a fraction of whole blood). A plasma fraction is any fraction that is produced by separation of one or more of the components of the plasma, by any means. For example, the plasma fraction may be a cryosupernatant which may e.g. be prepared by freezing the plasma, thawing it under controlled conditions and separating the liquid and solid components by centrifugation. A cryosupernatant may be further treated with solvent/detergent, subjected to chromatography such as cation exchange chromatography, anion exchange chromatography, and/or adsorption with various adsorbents to generate additional fractions.

Examples of plasma fractions therefore include platelet-rich plasma, a plasma concentrate, a precipitate from any fractionation of the plasma, a supernatant from any fractionation of the plasma, a cryoprecipitate, a cryosupernatant. Further examples include a fraction resulting from (i) solvent detergent treatment of a plasma cryosupernatant, (ii) solvent detergent treatment and/or cation exchange chromatography of a plasma cryosupernatant (e.g. solvent detergent treatment and cation exchange chromatography of a plasma cryosupernatant), and (iii) solvent detergent treatment and/or cation exchange chromatography and/or DEAE adsorption of a plasma cryosupernatant (e.g. solvent detergent treatment, cation exchange chromatography and DEAE adsorption of a plasma cryosupernatant).

The solution comprising anti-D immunoglobulin G which is subjected to the aluminium hydroxide gel adsorption step preferably comprises at least 0.2g/L IgG (e.g 0.2-1g/L, 0.3-0.6g/L IgG). In the solution comprising anti-D immunoglobulin G which is subjected to the aluminium hydroxide gel adsorption step preferably 60-80% of the IgG is of subtype IgG₁, and/or 10-20% of the IgG is of subtype IgG₃. Alternatively, or additionally, in the solution comprising anti-D immunoglobulin G which is subjected to the aluminium hydroxide gel adsorption step preferably 10-25% of the IgG is of subtype IgG₂ and/or 0.5-2% of the IgG is of subtype IgG₄. The solution comprising anti-D immunoglobulin G which is subjected to the aluminium hydroxide gel adsorption step preferably comprises 15-40µg/mL anti-D IgG (e.g 20-30µg/mL). The solution comprising anti-D immunoglobulin G which is subjected to the aluminium hydroxide gel adsorption step preferably comprises at least 20ng/ml, e.g. 20-200ng/ml, 50-150 ng/ml kallikrein-like activity. The solution comprising anti-D immunoglobulin G which is subjected to the aluminium hydroxide gel adsorption step preferably comprises at least 50nkat/L, e.g. 50-1000nkat/L, 100-5000 nKat/L serine protease activity. These activities are determined e.g. as described elsewhere herein using appropriate chromogenic substrates

### Aluminium hydroxide gel adsorption

The invention provides a step of aluminium hydroxide gel adsorption, which the inventors have devised in order to reduce protease activity (e.g. serine protease activity) in the solution comprising anti-D immunoglobulin G. Aluminium hydroxide gel (e.g. referred to as (Al(OH)₃) gel or Alhydrogel) is widely used as an adjuvant in view of its ability to improve attraction and uptake of antigen by antigen presenting cells (APCs). It is also used as an adsorbent.

In this step the solution comprising anti-D immunoglobulin G is contacted with aluminium hydroxide gel at a pH of about 4.70-4.90. By carrying out the aluminium hydroxide gel adsorption step at this pH, it is possible to reduce protease activity, without reducing the yield of anti-D immunoglobulin G.

Using other conditions, a reduction in protease activity can be achieved, but this is accompanied by a reduction in anti-D immunoglobulin G yield, and under other conditions still, the anti-D immunoglobulin G is not lost but the protease activity is not reduced (see Example 2). It was surprisingly found that carrying out the adsorption step within this pH range was the optimal way to balance the requirements of low protease activity and high anti-D immunoglobulin G yield.

The pH of the solution comprising anti-D immunoglobulin G may require adjustment prior to addition of the aluminium hydroxide gel, in which case this can be achieved by any suitable means. In one example an acid is used, which may be HCl (e.g. 0.2 M HCl).

In preferred embodiments the aluminium hydroxide gel is added to the solution comprising anti-D immunoglobulin G in an amount of 0.10-0.20 g/g protein, preferably 0.12-0.15 g/g protein, more preferably about 0.13 g/g protein.

In preferred embodiments the aluminium hydroxide gel treatment step occurs in a solution with a conductivity of 3.0-3.4 mS/cm, e.g. 3.1-3.3 mS/cm, preferably about 3.20 mS/cm.

Conductivity is the measure of an electrolyte solution's ability to conduct electricity. The SI unit of conductivity is Siemens per meter (S/m). The conductivity of a solution is generally measured, rather than calculated based on the concentration of the components. Conductivity is measured in units of S/cm but since the conductivity of a solution can vary depending on the temperature at which it is measured, unless otherwise stated, where conductivity values are referred to they are measured at 20-25°C (e.g. 20°C).

The contact of the mixture with aluminium hydroxide gel may be carried out for any suitable length of time, typically for as long as it takes to achieve effective protease activity reduction.

The method gives rise to a reduction in protease activity. By this it is meant that the solution that results from the contact with the aluminium hydroxide gel (e.g. in the liquid after separation of the liquid and solid components) has a lower protease activity than the solution before contact with the aluminium hydroxide gel. The proteases that contribute to the protease activity of the anti-D immunoglobulin G containing solution include serine proteases, e.g. kallikrein-like activity.

The activity of serine proteases may be measured by standard assays that are known in the art, e.g. using commercially available chromogenic substrates. An example of a suitable assay is an assay using the chromogenic substrate S-2288 (H-D-Ile-Pro-Arg-pNA *2HCl, available e.g. from Diapharma). Cleavage of S-2288 by serine proteases with arginine specificity gives rise to the production of pNA. The proteolytic activity is thus determined by p-nitroaniline (pNA) release. The formation of pNA can be followed spectrophotometrically at 405 nm, e.g. over a suitable time period. Suitable assays may be based on those described in [4]. The rate of formation of pNA can be compared to suitable controls having a known amount of proteolytic activity.

Kallikrein-like activity can also be measured by standard assays that are known in the art, e.g. using commercially available chromogenic substrates. An example of a suitable assay is an assay using the chromogenic substrate S-2302 (H-D-Pro-Phe-Arg-pNA ^{∗}2HCl, available e.g. from Diapharma). Cleavage of S-2302 by kallikrein-like activity gives rise to the production of pNA. The proteolytic activity is thus determined by p-nitroaniline (pNA) release. The formation of pNA can be followed spectrophotometrically at 405 nm, e.g. over a suitable time period. The rate of formation of pNA can be compared to suitable controls having a known amount of proteolytic activity.

In general, a reduction in protease activity as referred to herein results from the removal of the relevant protease(s) from the anti-D immunoglobulin G containing solution, e.g. as a result of the protease adsorbing to the aluminium hydroxide gel, rather than any inactivation of the relevant protease(s).

The reduction in protease activity is apparent in the solution resulting from the aluminium hydroxide gel step (e.g. in the liquid after separation of the liquid and solid components) and may be a reduction of at least 10, 20, 30, 40, 50, 60, 70, 80, 90 %. The reduction may be a reduction compared to the activity in the solution before it is contacted with the aluminium hydroxide gel.

There may also be a reduction in protease activity, e.g. a reduction of at least 10, 20, 30, 40, 50, 60, 70, 80, 90 % compared to the activity in a solution resulting from contacting identical starting solution with the aluminium hydroxide gel at a pH of 5.5, but using the same amount of the aluminium hydroxide gel on a g/g protein basis and at the same conductivity (e.g. under otherwise identical conditions).

In particular these reductions are seen when the aluminium hydroxide gel step is carried out on an anti-D immunoglobulin G containing solution that has been subjected to a solvent detergent treatment step as defined in more detail below, and in particular when the aluminium hydroxide gel step is carried out on an anti-D immunoglobulin G containing solution that has been subjected to a solvent detergent treatment step using Tri (n-butyl) phosphate (e.g. at 1 % (w/w)) and Polysorbate-20 (e.g. at 1.75% (w/w)).

The conditions are chosen to minimise the reduction in the yield of anti-D immunoglobulin G in the final product and hence also to minimise any loss of anti-D immunoglobulin G during the aluminium hydroxide gel step. Preferably the amount of anti-D immunoglobulin G in the anti-D immunoglobulin G containing solution after the aluminium hydroxide gel step (e.g. in the liquid after separation of the liquid and solid components) is at least 70, 75, 80, 85, 90, 95% of the amount in the anti-D immunoglobulin G containing solution before the aluminium hydroxide gel step. Preferably the amount of anti-D immunoglobulin G in the anti-D immunoglobulin G containing solution after the aluminium hydroxide gel step is at least 100, 110, 120% compared to the amount in a solution obtained from an identical starting solution but which has been contacted with aluminium hydroxide gel at a pH of 5.5, but using the same amount of aluminium hydroxide gel on a g/g protein basis and at the same conductivity of the amount in the anti-D immunoglobulin G containing solution (e.g. under otherwise identical conditions).

In typical embodiments, the contact is carried out for at least 10 minutes, e.g. between 10 minutes and 3 hours, 15 minutes and 2 hours, 20 minutes and 90 minutes, 30 minutes and 60 minutes and usually for about 45 to 55 minutes. The contact is conveniently carried out at about 15 to 25°C, e.g. about 18 to 23°C, 19 to 21°C or about 20°C.

In certain embodiments the mixture is stirred during the contact step. This may, for example be stirring at 100-500 rpm, e.g. 150-450, 200-400, 250-350, 300-325 rpm.

In certain embodiments one or more filter aids are added during the contact with the aluminium hydroxide gel, e.g. diatomaceous earth or perlite. Suitable examples include calcined diatomaceous earth (e.g. Celite). Optionally each of the one or more filter aids is added for at least 5, 10, 15, 20, 25, 30, 35, 50 minutes. If more than one filter aid is added they may be added together or sequentially. The filter aid(s) may be added after an initial period of contact with the aluminium hydroxide gel alone, for example, the aluminium hydroxide gel is added to the anti-D immunoglobulin G containing solution and one or more filter aid is added after a period of time, e.g. after at least 10, 15, 20, 25, 30, 35, 50 minutes, e.g. for a period of at least 5, 10, 15 minutes. Filter aids having a permeability of between 0.050-0.085 Da can optionally be used, e.g Celite 574, Celite 545, Filter Cel C505 and C505CZ. A mixture comprising one of more of the filter aids may optionally be used. The mixture may comprise Celite 574. The mixture may comprise Celite 545.

In some embodiments, the filter aid Celite 574 is used in the amount: Celite 574 [g] = weight DEAE filtrate [g] x 0.00025.

In some embodiments, the filter aid Celite 545 is used in the amount: Celite 545 [g] = weight DEAE filtrate [g] x 0.00025.

The solid and liquid components of the mixture are then separated by any means known in the art, for example centrifugation or filtration. The liquid component is a further anti-D immunoglobulin G containing solution.

In certain embodiments, the unbound anti-D IgG fraction is filtered through a mesh e.g. a polypropylene mesh, to separate solid and liquid components.

In certain embodiments, a filter cake is formed on top of a mesh using an appropriate amount of the needed Celite e.g. half the amount of the needed Celite, in a small volume of the appropriate buffer.

The remaining amount of Celite is then mixed into the immunoglobulin-Alhydrogel suspension, immediately before filtering.

Filter aids are particularly useful where an anion exchange chromatography step is included prior to aluminium hydroxide gel adsorption.

### Solvent detergent virus inactivation step

The process of the invention may involve inactivating one or more viruses. Solvent detergent treatment results in inactivation of one or more viruses that may be in the solution. The viruses will typically be contaminating viruses that enter the solution from the environment or were present in material from which the solution was made. The presence or absence of the viruses in the solution may not be known, and so the treatment is in general used to reduce the risk of viral contamination by inactivating one or more viruses that may be in the solution. When viruses are present in the solution, the incubation results in inactivation of one or more of these viruses. Solvent detergent treatment is preferably carried out before the aluminium hydroxide gel step.

In one aspect of the invention the solvent used in the solvent detergent treatment step may be Tri (n-butyl) phosphate. This is preferably used at 0.5-1.5%, e.g. 0.75-1.25%, preferably 1 % (w/w).

In one aspect of the invention the detergent used in the solvent detergent treatment step may be Polysorbate-20. This is preferably used at greater than 1% and up to 2% (w/w) e.g. about 1.1% (w/w), about 1.2% (w/w), about 1.3% (w/w), about 1.4% (w/w), about 1.5 % (w/w), about 1.6% (w/w), about 1.7 % (w/w), about 1.8 % (w/w), or about 1.9 % (w/w), or 1.2-1.9 % (w/w), 1.5-1.8 % (w/w), 1.7-1.8% (w/w). Particularly preferred amounts are 1.70, 1.71, 1.72, 1.73, 1.74, 1.75 or 1.76 (w/w), with 1.75% (w/w) being particularly preferred.

Conditions for solvent detergent treatment for viral inactivation are well known in the art. In one embodiment the solvent detergent treatment step involves incubation of the solvent and detergent with the anti-D IgG containing material for at least 10, 20, 30, 40, 50, 60, 70, 80, 90, 100, 120 minutes, more preferably at least 60 minutes. This step preferably occurs at 25-35°C, e.g. 26-34°C, 27-33°C, 28-32°C, 29-31°C, or around 30°C. The anti-D IgG containing material may be brought to the required temperature before adding the solvent and detergent. The mixture may be stirred during the incubation, optionally at 100-500 rpm, e.g. 150-450, 200-400, 250-350, 300-325 rpm.

The solvent detergent treatment step may involve a second incubation for at least 90, 100, 120, 150, 180, 210, 240, 270, 300, more preferably at least 240minutes. This step preferably occurs at 25-35°C, e.g. 26-34°C, 27-33°C, 28-32°C, 29-31°C, or around 30°C. The mixture may be stirred during the incubation, optionally at 100-500 rpm, e.g. 150-450, 200-400, 250-350, 300-325 rpm. When this second incubation is carried out the product of the first incubation may be filtered prior to the second incubation stage. This filtration may be carried out using a micron-sized filter. Suitable filters include filters of 0.5µm or less, e.g. a 0.45, 0.3, 0.2, or 0.1 µm filter. Preferably the filter is a 0.45 µm filter. The product of the first incubation may first be passed through a pre-filter. Suitable pre-filters include micron-sized filters, e.g. filters of 1µm or 0.5µm or less, e.g. a 0.45, 0.3, 0.2, or 0.1 µm filter.

The virus-inactivated solution is left without stirring for at least 600 minutes (e.g. 6001500, 720-1440, 780-1380, 840-1320, 840-1260 or 840-1200 minutes, at 35-445C, preferably 37-43C, more preferably 39 - 41 °C for phase separation. The lower, non-lipid (lipid-poor) phase contains the anti-D IgG and is processed further (e.g. it is a further anti-D immunoglobulin G containing solution).

### Cation exchange step

The process of the invention may involve a step of cation exchange chromatography. This may occur after the solvent detergent treatment step (e.g. it may be carried out on an anti-D immunoglobulin G containing solution which has been subjected to solvent detergent treatment). In cation exchange chromatography, positively charged molecules are attracted to a negatively charged solid support. Any negatively charged ligand attached to the solid phase suitable to form the cation exchange matrix can be used, *e.g.* a carboxylate, sulfonate and others as described below. Commercially available cation exchange matrices include, but are not limited to, for example, those having a sulfonate based group (*e.g*. MonoS^{®}, MiniS, Source^{™} 15S and 30S, SP Sepharose^{®} Fast Flow^{™}, SP Sepharose^{®} High Performance from GE Healthcare, Toyopearl^{®} SP-650S and SP-650M from Tosoh, Macro-Prep^{®} High S from BioRad, Ceramic HyperD^{®} S, Trisacryl^{®} M and LS SP and Spherodex^{®} LS SP from Pall Technologies; a sulfoethyl based group (*e.g*. Fractogel^{®} SE from EMD Millipore, POROS^{®} (S-10 and S-20 from ThermoFisher); a sulphopropyl based group (*e.g*. TSK Gel^{®} SP 5PW and SP-5PW-HR from Tosoh, POROS^{®} HS-20 and HS 50 from ThermoFisher); a sulfoisobutyl based group (*e.g*. Fractogel^{®} EMD SO3 from EMD Millipore); a sulfoxyethyl based group (*e.g*. SE52, SE53 and Express-Ion^{™} S from Whatman), a carboxymethyl based group (*e.g*. CM Sepharose^{®} Fast Flow from GE Healthcare, Hydrocell CM from Biochrom Labs Inc., Macro-Prep^{®} CM from BioRad, Ceramic HyperD^{®} CM, Trisacryl M CM, Trisacryl LS CM, from Pall Technologies, Matrex Cellufine C500 and C200 from Millipore, CM52, CM32, CM23 and Express - Ion^{™} C from Whatman, Toyopearl^{®} CM-650S, CM-650M and CM-650C from Tosoh); sulfonic and carboxylic acid based groups (*e.g*. BAKERBOND^{®} Carboxy-Sulfon from J.T. Baker); a carboxylic acid based group (*e.g*. WP CBX from J.T Baker, DOWEX^{®} MAC-3 from Dow Liquid Separations, Amberlite^{™} Weak Cation Exchangers, DOWEX^{®} Weak Cation Exchanger, and Diaion Weak Cation Exchangers from Sigma-Aldrich and Fractogel^{®} EMD COO- from EMD); a sulfonic acid based group (*e.g*. Hydrocell SP from Biochrom Labs Inc., DOWEX^{®} Fine Mesh Strong Acid Cation Matrix from Dow Liquid Separations, UNOsphere^{®} S, WP Sulfonic from J. T. Baker, Sartobind^{®} S membrane from Sartorius, Amberlite^{™} Strong Cation Exchangers, DOWEX^{®} Strong Cation and Diaion Strong Cation Exchanger from Sigma-Aldrich); and a orthophosphate based group (*e.g.* PI 1 from Whatman).

If desirable, a cation exchange membranes or cation exchange matrices, *e.g*. Sartobind^{®} S (Sartorius; Edgewood, NY) may be used.

The cation exchange step preferably uses a weak cation exchanger, such as one based on ligands having a carboxymethyl group. The cation exchange matrix may comprise methacrylate beads (*e.g* methyacrylate beads comprising ligands having a carboxymethyl group). In certain embodiments the cation exchange step uses a chromatography column comprising ligands possessing a carboxymethyl group. The column may optionally be equilibrated with a suitable equilibration buffer (e.g. a phosphate buffer). The material (e.g. an anti-D immunoglobulin G containing solution, such as a cryosupernatant which has undergone solvent detergent treatment) is applied to the column (optionally after dilution e.g. in the equilibration buffer), and optionally the column is washed with a suitable washing buffer. This is followed by an elution step. The eluted material is a further anti-D immunoglobulin G containing solution. Exemplary conditions for these steps are as follows: dilution of the anti-D immunoglobulin G containing solution to 3.00 - 3.40 mS/cm, with a concentration of 8 - 12 g/L and a pH of 5.45 -5.55. Exemplary equilibration buffer has a conductivity of 3.00 - 3.40 mS/cm and a pH of 5.45 -5.55. When dilution of the material is carried out the diluted material may be filtered prior to being applied to the column. This filtration may be carried out using a micron-sized filter. Suitable filters include filters of 0.5µm or less, e.g. a 0.45, 0.3, 0.2, or 0.1 µm filter. Preferably the filter is a 0.45 µm filter.

In certain embodiments the cation exchange step uses a chromatography column comprising a carboxymethyl based group. The column may be equilibrated with equilibration buffer comprising sodium phosphate buffer (e.g. 50 mM sodium phosphate buffer, pH 5.5, 3.2 mS/cm). The material (e.g. an anti-D immunoglobulin G containing solution) is applied to the column in the equilibration buffer and washed with a washing buffer (e.g. a 25 mM sodium phosphate buffer, pH 7.0). This is followed by an elution step. The elution step may use sodium phosphate buffer and sodium chloride (e.g. 25 mM sodium phosphate buffer + 0.2 M sodium chloride, pH 7.5).

### Anion exchange step

Further purification steps may be included in the process, either before the step of aluminium hydroxide gel adsorption, or after. Each further purification step may be a chromatography and/or an adsorption step, *e.g*. immunoaffinity, affinity, hydrophobic interaction, ion exchange, multimodal, size exclusion or metal chelate chromatography/adsorption.

For example, an ion exchange step may be carried out on the solution before the step of aluminium hydroxide gel adsorption (e.g. after a cation exchange step, or after a solvent detergent treatment step, or after a solvent detergent treatment step and a cation exchange step). Anion exchange using DEAE (as described below) is particularly suitable for this step.

In anion exchange chromatography, negatively charged molecules are attracted to a positively charged solid support, e.g. a resin or gel. A positively charged solid support can be prepared by any means known to persons skilled in the art and will usually involve the covalent attachment of a positively charged functional ligand onto a solid support. Suitable positively charged functional ligands will invariably depend on the material to be separated from the solution. Examples of suitable anion exchange resins or gels are ones comprising a functional quaternary amine group (Q) and/or a tertiary amine group (DEAE), or a diethylaminopropyl group (ANX). Commercially available anion exchange chromatography matrices include, but are not limited to, DEAE cellulose, Poros^{™} PI 20, PI 50, HQ 10, HQ 20, HQ 50, D 50, XQ from ThermoFisher, MonoQ^{™}, MimQ^{™}, Source^{™} 15Q and 30Q, Q, DEAE and ANX Sepharose Fast Flow^{™}, Q Sepharose high Performance^{™}, QAE SEPHADEX^{™} and FAST Q SEPHAROSE^{™} from GE Healthcare, WP PEI^{™}, WP DEAM^{™}, WP QUAT^{™} from J.T. Baker, Hydrocell^{™} DEAE and Hydrocell^{™} QA from Biochrom Labs Inc., UNOsphere^{™} Q, Macro-Prep^{™} DEAE and Macro-Prep^{™} High Q from Biorad, Ceramic HyperD^{™} Q, ceramic HyperD^{™} DEAE, Q HyperZ^{™}, Trisacryl^{™} M and LS^{™} DEAE, Spherodex^{™} LS DEAE, QMA Spherosil^{™} LS, QMA Spherosil^{™} M from Pall Technologies, DOWEX^{™} Fine Mesh Strong Base Type I and Type II Anion Matrix and DOWEX^{™} MONOSPHER E 77, weak base anion from Dow Liquid Separations, Matrex Cellufme^{™} A200, A500, Q500, and Q800, from Millipore, Fractogel^{™} EMD TMAE₃ Fractogel^{™} EMD DEAE and Fractogel^{™} EMD DMAE from EMD, Amberlite^{™} weak and strong anion exchangers type I and II, DOWEX^{™} weak and strong anion exchangers type I and II, Diaion^{™} weak and strong anion exchangers type I and II, Duolite^{™} from Sigma-Aldrich, TSK^{™} gel Q and DEAE 5P and 5PW-HR, Toyopearl^{™} SuperQ-650S, 650M and 650C₃ QAE-26- 550C and 650S, DEAE- 650M and 650C from Tosoh, and QA52^{™}, DE23^{™}, DE32^{™}, DE51^{™}, DE52^{™}, DE53^{™}, Express-Ion^{™} D and Express-Ion^{™} Q from Whatman. DEAE is preferred.

If desirable, an anion exchange chromatography membrane can be used. Commercially available anion exchange membranes include, but are not limited to, Sartobind^{™} Q from Sartorius, Mustang^{™} Q from Pall Technologies and Intercept^{™} Q membrane from Millipore.

In one aspect of the invention the anion exchange medium used in the anion exchange step is a gel with a weak anion exchanger, e.g. DEAE as the functional group. A suitable base matrix may be beads made of cross-linked dextran, and an example of a suitable anion exchange medium is DEAE Sephadex.

The DEAE containing gel is contacted with the material (e.g. an anti-D immunoglobulin G containing solution), and the solid and liquid components are subsequently separated by any appropriate means (e.g. by filtration or centrifugation). The liquid component is a further anti-D immunoglobulin G containing solution.

### Cation exchange step after aluminium hydroxide gel step

As discussed above, purification of anti-D immunoglobulin G is typically performed by carrying out one or more steps of chromatography on an anti-D immunoglobulin G containing solution. The chromatography may be selected from any suitable chromatography, e.g. immunoaffinity, affinity, hydrophobic interaction, ion exchange, multimodal, size exclusion or metal chelate chromatography.

The process of the invention may involve a chromatography step after the aluminium hydroxide gel adsorption step. This chromatography step may use ion exchange, preferably cation exchange. Suitable cation exchange materials and conditions are discussed above.

The cation exchange step may use a chromatography column equilibrated with equilibration buffer comprising sodium phosphate buffer (e.g. 50 mM sodium phosphate buffer, pH 5.5). The material (e.g. an anti-D immunoglobulin G containing solution) is applied to the column in the equilibration buffer and washed with a washing buffer (e.g. a 25 mM sodium phosphate buffer, pH 5.45-5.55, conductivity 3.0-3.4mS/cm)|. This is followed by an elution step. The elution step may use sodium phosphate buffer and sodium chloride (e.g. 25 mM sodium phosphate buffer + 0.2M sodium chloride, pH 5.5).

Optionally the anti-D immunoglobulin G containing solution is filtered before it is applied to the cation exchange chromatography column, e.g. using a micron filter. Suitable sizes include filters of 1µm or 0.5µm or less, e.g. a 0.45, 0.3, 0.2, or 0.1 µm filter.

In a preferred embodiment the process comprises the steps of: a) providing plasma from human Rh-negative donors who have been sensitised to rhesus factor D or a fraction thereof, comprising anti-D immunoglobulin G; b) carrying out a solvent detergent virus inactivation step on the plasma or fraction thereof using Tri (n-butyl) phosphate (TnBP) and Polysorbate-20 (s); c) carrying out a cation exchange chromatography step on the solvent detergent treated plasma or fraction thereof; d) carrying out an anion exchange step on the eluate from step c); d) contacting said solution with aluminium hydroxide gel in an amount of 0.10-0.20g/g protein at a pH of 4.70 to 4.90 and a conductivity of 3.0 to 3.4 mS/cm ; and separating the solution from the solid components; e) carrying out a further step of cation exchange chromatography on the solution from d).

### Virus filtration

The method may further comprise a step of viral filtration, e.g. after one or more of a solvent detergent virus inactivation step, a cation exchange chromatography step, an anion exchange step, an aluminium hydroxide gel step, a cation exchange chromatography step. In certain aspects of the invention, the solution comprising the anti-D immunoglobulin G from the preceding step is subjected to filtration for the removal of viral particles, including intact viruses, and microbes, if present. For example, viral filtration membranes of pore sizes from 15-20 nm may be used to remove microbes and viruses from a solution or eluate or pharmaceutical composition. Exemplary nanofilters include Planova S20N (Asahi), Virosart HC (Sartorius), Planova 15N (Asahi) and 20N (Asahi). Other viral filters can be used in this filtration step and are well known to those skilled in the art.

In certain embodiments, following the filtration process, the filter is washed using *e.g*. the elution buffer used in the preceding step, in order to remove any anti-D immunoglobulin G retained in the filter housing. The viral filter may e.g. be a hollow fibre filter, e.g. a PVDF (polyvinylidene fluoride) hollow fibre filter.

In a preferred embodiment the process comprises the steps of: a) providing plasma from human Rh-negative donors who have been sensitised to rhesus factor D or a fraction thereof, comprising anti-D immunoglobulin G; b) carrying out a solvent detergent virus inactivation step on the plasma or fraction thereof using Tri (n-butyl) phosphate (TnBP) and Polysorbate-20 (PS-20); c) carrying out a cation exchange chromatography step on the solvent detergent treated plasma or fraction thereof; d) carrying out an anion exchange step on the eluate from step c); d) contacting said solution with aluminium hydroxide gel in an amount of 0.10-0.20g/g protein at a pH of 4.70 to 4.90 and a conductivity of 3.0 to 3.4 mS/cm ; and separating the solution from the solid components; e) carrying out a further step of cation exchange chromatography on the solution from d); f) filtering the solution from e) to reduce the amount of any virus present.

Optionally the method further comprises g) one or more of (i) formulating the solution, optionally with glycine and/or albumin, and (ii) filling syringes with the solution.

### Protease activity of the final preparation

The process of the invention may provide a solution of anti-D immunoglobulin G following the above described steps. The level of the protease activity in any step following the aluminium hydroxide gel step is reduced as described above, when compared to the level of protease activity before the aluminium hydroxide gel step.

The process of the invention may provide a solution of anti-D immunoglobulin G comprising a level of protease activity that is less than 50 nkat/1 , optionally less than 40, 30, 20, 15 nkat/1 and/or a kallikrein- like activity that is less than 40 ng/ml, optionally less than 30, 30, 20, 15 ng/ml. Typically, the level of protease activity is less than 20 nkat/1 and/or the kallikrein- like activity is less than 20 ng/ml. Such levels can be determined e.g. by using the chromogenic substrate assays, e.g. as described above.

### Amount of anti-D immunoglobulin G in the final preparation

The process of the invention may provide a solution following the purification of the treated plasma or fraction thereof comprising 1-5g/L (e.g. 1.5-5g/L, 2-4g/L) immunoglobulin G and/or 50-200µg/ml (e.g. 75-175µg/ml, 100-150µg/ml) anti-D immunoglobulin G. Typically, the amount of immunoglobulin Gis 1-2g/L and/or the amount of anti-D immunoglobulin Gis 100-150µg/ml.

### Pharmaceutical compositions and methods

Pharmaceutical compositions of the invention can be prepared for storage as a solution by mixing the anti-D immunoglobulin G solution with optional pharmaceutically-acceptable carriers, excipients or stabilizers typically employed in the art (all of which are referred to herein as "carriers"), *i.e.* buffering agents, stabilizing agents, preservatives, isotonifiers, non-ionic detergents, antioxidants, and other miscellaneous additives (see [5]). Such additives must be nontoxic to the recipients at the dosages and concentrations employed. By way of example albumin and/or an amino acid such as glycine may be added to the formulation. The compositions may be prepared in various forms. For example, the compositions may be prepared as injectables.

The pharmaceutical composition is typically sterile. It is preferably pyrogen-free.

In many embodiments, the composition is buffered *e.g*. at between pH 5 and pH 6, generally around pH 5.15-5.25.

The invention also provides a delivery device containing a pharmaceutical composition of the invention. The device may be, for example, a syringe.

In a preferred embodiment the process comprises the steps of: a) providing plasma from human Rh-negative donors who have been sensitised to rhesus factor D or a fraction thereof, comprising anti-D immunoglobulin G; b) carrying out a solvent detergent virus inactivation step on the plasma or fraction thereof using Tri (n-butyl) phosphate (TnBP) and Polysorbate-20 (PS-20); c) carrying out a cation exchange chromatography step on the solvent detergent treated plasma or fraction thereof; d) carrying out an anion exchange step on the eluate from step c); d) contacting said solution with aluminium hydroxide gel in an amount of 0.10-0.20g/g protein at a pH of 4.70 to 4.90 and a conductivity of 3.0 to 3.4 mS/cm ; and separating the solution from the solid components; e) carrying out a further step of cation exchange chromatography on the solution from d); f) filtering the solution from e) to reduce the amount of any virus present; g) one or more of (i) formulating the solution, optionally with glycine and/or albumin, and (ii) filling syringes with the solution.

Once formulated, the compositions of the invention can be administered directly to a subject. The subjects to be treated can be animals; in particular, human subjects can be treated.

### General

The term "comprising" encompasses "including" as well as "consisting" *e.g*. a composition "comprising" X may consist exclusively of X or may include something additional *e.g*. X + Y.

The term "about" in relation to a numerical value x means, for example, *x*±10%.

The word "substantially" does not exclude "completely" *e.g*. a composition which is "substantially free" from Y may be completely free from Y. Where necessary, the word "substantially" may be omitted from the definition of the invention.

Where the invention provides a process involving multiple sequential steps, the steps are carried out in the indicated order, *i.e.* in numerical or alphabetical order. However, the skilled person will understand that the order of steps may be altered while still achieving useful results. The invention can also provide a process involving less than the total number of steps.

### BRIEF DESCRIPTION OF DRAWINGS

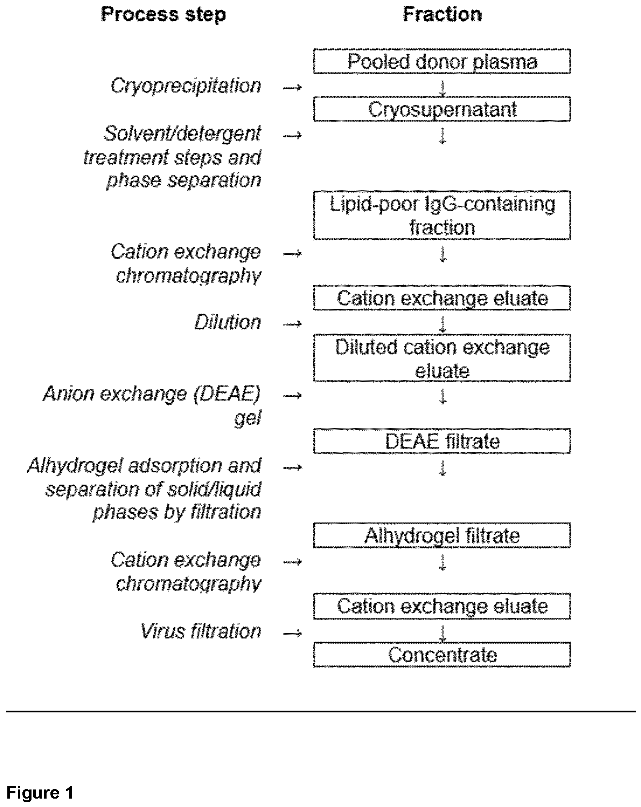

Figure 1 shows an overview of the process for preparing anti-D immunoglobulin
Figure 2 shows the design of an experiment to optimize proteolytic activity depletion during aluminium hydroxide gel treatment.
Figure 3 shows proteolytic and kallikrein-like activity of two process intermediates referred to as 1.11 (the aluminium hydroxide gel filtrate) and 1.14 (the formulated bulk).
Figure 4 shows anti-D recovery over the aluminium hydroxide gel step and the entire process.

### MODES FOR CARRYING OUT THE INVENTION

### Example 1

### Testing for contaminants in downscale process

A downscale protocol for the process for anti-D production using Triton X-100 was devised in order to compare this process with potential new processes and to further test new parameters. The downscale protocol was first validated by comparing levels of various components, including Anti-D, IgG, IgA, at different stages of the process with levels of each component according to historical data from manufacturing lots. Once validated, the downscale protocol was then used to test PS-20 as an appropriate alternative detergent to T100 in the solvent detergent step, as well as identifying suitable conditions for a solvent detergent step including PS-20.

One of the outcomes of this analysis, in which components were detected in the downscale protocol starting material (cryo-poor plasma), the feed for the first cation exchange (before dilution), the DEAE filtrate, the aluminium hydroxide gel filtrate and the formulated bulk, was to identify proteolytic activity as a quality attribute. Certain other plasma components showed elevated levels immediately after the solvent detergent step, but these returned to acceptable values by the time the bulk material was generated. It was therefore desirable to seek to further reduce the proteolytic activity and the kallikrein-like activity both of which were elevated after a solvent detergent treatment using PS-20.

Proteolytic activity was measured using the chromogenic substrate S-2288 (H-D-Ile-Pro-Arg-pNA *2HCl, available e.g. from Diapharma), activity being determined by p-nitroaniline (pNA) release. The formation of pNA can be followed spectrophotometrically at 405 nm, e.g. over a suitable time period. Suitable assays may be based on those described in [4].

Kallikrein-like activity was measured using the chromogenic substrate S-2302 (H-D-Pro-Phe-Arg-pNA ^{∗}2HCl, available e.g. from Diapharma), with cleavage again being determined by pNA release. The formation of pNA can be followed spectrophotometrically at 405 nm, e.g. over a suitable time period.

The rate of formation of pNA can be compared to suitable controls having known amounts of proteolytic and/or kallikrein-like activity.

### Example 2

### Reduction of pH during aluminium hydroxide gel treatment depletes proteolytic activity

### Summary

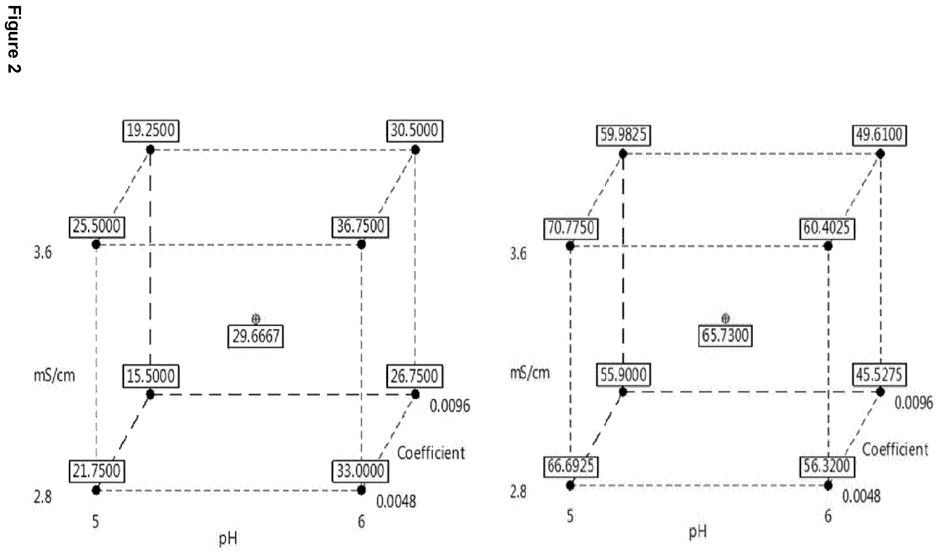

As the downscale process with PS-20 led to slightly elevated levels of proteolytic activity (protease activity as assessed by release of pNA from chromogenic substrate S-2288 and kallikrein-like activity as assessed by release of pNA from chromogenic substrate S-2302), compared to the process when T100 was used in the solvent detergent step of the process, the ability of the aluminium hydroxide gel treatment step to mitigate these elevated levels was investigated. In this study, aluminium hydroxide gel amount, pH and conductivity were varied and optimized for the depletion of proteolytic activity without anti-D loss (Fig. 2).

### Method and results

In a comparative process, the following values were used: Al(OH)₃ solution amount = 0.0048, pH = 5.5 and conductivity = 3.2. This solution amount is calculated depending on the weight and protein content of DEAE filtrate to give a value of 0.1324g/g of protein (amount of 2% Alhydrogel solution [g] = weight DEAE filtrate [g] x OD280 [AU] x 0.0048). In this experiment, parameters were tested across the following ranges: 2 % Al(OH)₃ solution amount = coefficient from 0.0048 to 0.0096 (corresponding to 0.1324 and 0.2648 g / g protein), pH = from 5 to 6 and conductivity = from 2.8 to 3.6.

Nine conditions for Aluminium hydroxide gel adsorption were set up in parallel as shown in Figure 2. The starting material was the filtrate from the DEAE adsorption step of the P-20 downscale process. This was contacted with Al(OH)₃ solution for 30 minutes, following which Celite 574 was added. After 5 more minutes Celite 545 was added for 15 minutes (total 50 minutes). After separation of the solid and liquid components the proteolytic activity and anti-D recovery were measured. Proteolytic activity (left-hand box in Fig 2, nkat/L) was measured as referred to above. Anti-D recovery (right-hand box in Fig 2, %) was measured by a flow cytometry based method. The method is one of three methods permitted by Ph. Eur to determine anti-D immunoglobulins quantitatively in which the specific binding of a fluorescence-labeled antibody to cell-bound anti-D immunoglobulin G is determined and compared to a reference standard [6]. The obtained values were plotted. As expected, an increased amount of Al(OH)₃ led to a better depletion of proteolytic activity, however this also led to an undesirable loss of anti-D. As expected too, a decreased conductivity led to a better depletion of proteolytic activity, however also an undesirable loss of anti-D. However, surprising it was found that a decrease in pH led to a better depletion of proteolytic activity without a loss of anti-D.

Downscale bulks were then produced with lowered pH, during Al(OH)₃ adsorption. Proteolytic activity and kallikrein-like activity were measured in two process intermediates referred to as 1.11 (the aluminium hydroxide gel filtrate) and 1.14 (the formulated bulk).

The use of a lower pH at the aluminium hydroxide gel stage resulted in a more efficient depletion of the proteolytic activity and kallikrein-like activity in these process intermediates.

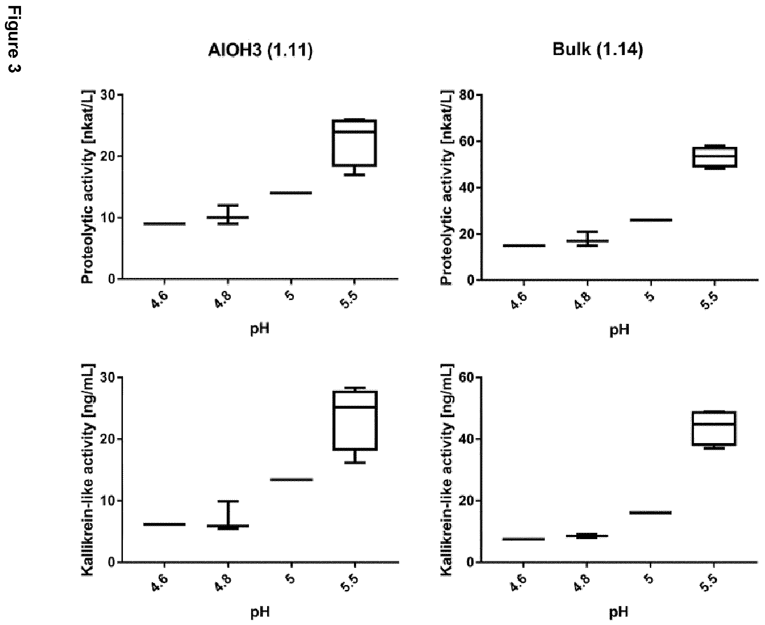

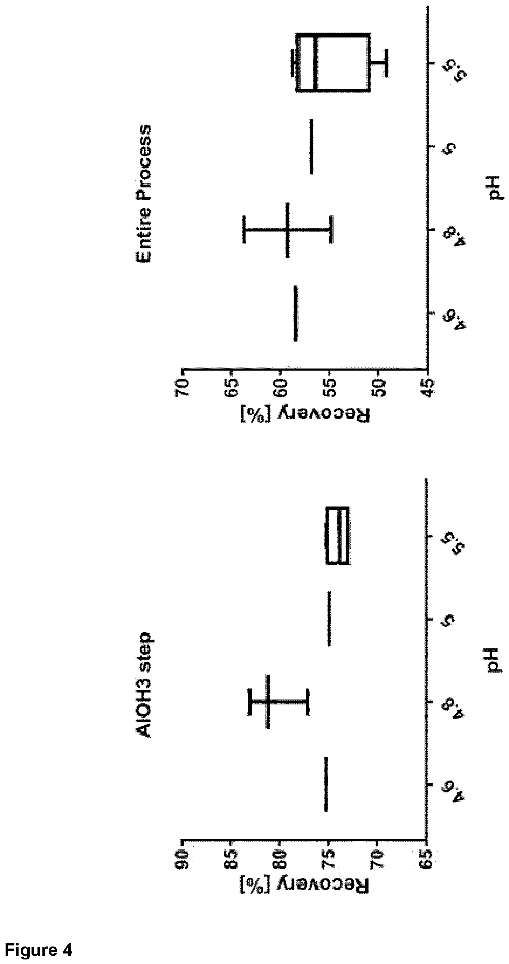

Figure 3 shows that the proteolytic and kallikrein-like activity of process intermediates 1.11 and 1.14 decreased when downscale bulks were produced with different pHs for the aluminium hydroxide gel treatment. It was confirmed that a lowered pH led to a depletion of proteolytic and kallikrein-like activity of these process intermediates. At a pH of 4.8 the proteolytic activity was depleted 2-3 times more efficiently than at pH 5.5. This additional depletion is interesting because it shows that protease activity can be removed to a greater extent using the conditions identified by the inventors. At pH 4.8 anti-D recovery was optimal both over the entire process and the aluminium hydroxide gel step (Fig. 4).

### Conclusion

Anti-D recovery was optimal both over the entire process and the aluminium hydroxide gel step at pH 4.8. Thus, the aim to reduce the proteolytic activity in the P20 downscale process to a similar level as in the T100 process without anti-D loss was achieved by carrying out the aluminium hydroxide gel treatment at pH 4.8.

### Example 3

### Scale up runs to assess factors including product- related impurities

The process was then scaled up in order to generate sufficient anti-D for comprehensive analysis and stability studies. The scale up runs produced approximately 400ml final bulk of two P20 lots for analysis (referred to as A and B), which was compared to one T100 run (C). Intermediates and bulk were tested for the presence of various product-related impurities at various stages including the following: scale up run starting material (cryo-poor plasma), the filtered mixture after the first SD treatment stage (referred to as FT3) the CM1 eluate (referred to as CM1), the DEAE filtrate (referred to as DEAE), the aluminium hydroxide gel filtrate (referred to as AlOH₃) and the formulated bulk. The values obtained were compared to known values from the current manufacturing process.

### Kallikrein-like activity

The kallikrein-like activity was measured using a chromogenic assay. The kallikrein-like activity is increased after phase separation in FT3 (Table 1), with higher values for the PS-20 based process. In this process, kallikrein-like activities at stages CM1 and DEAE exceed the recommended 3-σ limits, however, are reduced during the optimized Al(OH)₃ treatment to levels below the average activity observed in MFG. Kallikrein-like activity in the aluminium hydroxide gel filtrate (Al(OH)₃) and bulk are higher in the new compared to the reference process, but comparable to downscale experiments performed during development, indicating a robust depletion of kallikrein, independent of the manufacturing scale. Moreover, the values derived from the reference process are unusually low compared to the kallikrein-like activity observed in the control experiments that were carried out during the development of the reference process. This is in accordance with the observation that protease activities are in general lower in lab-scale compared to MFG, which may arise as a result of temperature differences in the manufacturing process at the phase separation stage. Consequently, protease activities are propagated in the downstream process steps, resulting in increased activities in MFG compared to the lab-scale experiments performed at target temperature. No relevant difference to the absolute values is observed, when looking at the kallikrein-like activities normalized to the protein content (ng/mg protein, not shown in Table).

**Table 1**

| | **Current Process** | | | **New Process** | |
|---|---|---|---|---|---|
| **Kallikrein-like activity [ng/mL]** | **Recommended 3-σ limit** | **MFG** | **C** | **A** | **B** |
| **Cryo Sup 1-2** | ≤ 133 | n/a | n/a | 29 | 38 |
| **Cryo Filtrate 1-4** | ≤ 113 | 72 ± 25 | 24 | n/a | n/a |
| **FT3** | n/a | n/a | 707 | 4497 | 6339 |
| **CM1** | ≤ 1813 | 1186 ± 97 | 877 | 1907 | 1931 |
| **DEAE** | ≤ 67 | 55 ± 5 | 32 | 108 | 94 |
| **Al(OH)₃** | ≤ 28 | 24 ± 2 | β | 17 | 19 |
| **Bulk** | ≤ 41 | 30 ± 5 | 3 | 9 | 10 |

### Protease activity at optimal pH for serine proteases

The protease activity was measured using a chromogenic assay described above. The same tendencies are observed as for kallikrein-like activity although there is variability between the two experiments at stage CM1 of the new process (Table 2). This was carried out at optimal pH.

**Table 2**

| | **Current Process** | | | **New Process** | |
|---|---|---|---|---|---|
| **Proteolytic activity [nkat/L]** | **Recommended 3-σ limit** | **MFG** | **C** | **A** | **B** |
| **Cryo Sup 1-2** | ≤ 476 | n/a | n/a | 218 | 275 |
| **Cryo Filtrate 1-4** | ≤ 1724 | 249 ± 103 | 245 | n/a | n/a |
| **FT3** | n/a | n/a | 2443 | 5610 | 5621 |
| **CM1** | ≤ 4883 | 3293 ± 327 | 4370 | 6385 | 3411 |
| **DEAE** | ≤ 264 | 172 ± 20 | 126 | 411 | 455 |
| **Al(OH)₃** | ≤ 34 | 27 ± 20 | 8 | 9 | 22 |
| **Bulk** | ≤ 54 | 41 ± 5 | 5 | 17 | 14 |

### Example 4

### Outline of the new production process

The starting material is plasma of Rh-negative individuals who have been sensitized to rhesus factor D. The plasma obtained through plasmapheresis is subjected to cryofractionation.

### Solvent detergent treatment using PS-20

The cryo-poor plasma is treated with 1.75% PS-20 and 1% tri(n-butyl) phosphate for at least one hour at 30°C with mixing. The mixture is filtered through a 0.45 µm filter. The second stage of solvent detergent treatment involves incubation for virus inactivation for ≥ 240 minutes at 30°C with mixing. The virus-inactivated solution is left standing for lipid phase separation for ≥ 13 h at ≥ 37 °C. The lipid-poor solution containing the IgG is separated for further processing.

### Cation Exchange Chromatography ("CM1")

The material is diluted with buffer and filtered through a micron-sized filter prior to being loaded onto a weak cation exchanger (carboxymethyl (CM) resin).

The column is washed with 25 mM sodium phosphate buffer, pH 7.0, and the IgG fraction containing anti-D is eluted with a solution containing 25 mM sodium phosphate buffer and 0.2 M sodium chloride, pH 7.5.

### Anion Exchange Chromatography ("DEAE")

The eluate from the CM step is diluted with water to a conductivity of 3.3 mS/cm, the pH is adjusted to 7.5 with NaOH, and passed through DEAE-Sephadex^{®} A50 dry resin (Pharmacia, Uppsala, Sweden). The unbound anti-D IgG fraction is filtered through a polypropylene mesh and the pH is adjusted to 4.8 with 0.2 M HCl.

### Alhydrogel Adsorption

The DEAE filtrate is treated with 0.13 g of Al(OH)₃ gel per g protein and stirred for 30 minutes at 20-25°C. This is followed by the addition of Celite 574 (Celite 574 [g] = weight DEAE filtrate [g] x 0.00025) stirred for 5 minutes and then Celite 545 (Celite 545 [g] = weight DEAE filtrate [g] x 0.00025) and stirred for 15 minutes. Different filter aids were tested for the removal of Alhydrogel e.g. Perlite J 100, Celite 574, Celite 545, Celite 503, and Diacel 150. It was found that a mixture of Celite 574 and Celite 545 gave best results with respect to flow rate, retention of Alhydrogel, and recovery of Anti-D IgG. The unbound anti-D IgG fraction is filtered through a polypropylene mesh to separate solid and liquid components.

In an alternative method, a filter cake is formed on top of a polypropylene mesh using half of the needed Celite in a small volume of the appropriate buffer. The remaining amount of Celite is then mixed into the immunoglobulin-Alhydrogel suspension, immediately before filtering.

### Cation Exchange Chromatography ("CM2")

To increase anti-D concentration, the DEAE filtrate is bound a second time to a CM resin. The IgG fraction containing anti-D is eluted with 25 mM sodium phosphate buffer + 0.2 M sodium chloride, pH 5.5.

### Virus filtration

This may be performed on the eluate using a nanofilter, such as the PLANOVA Nanofilter 15N.

### REFERENCES

[1] WO9518155
[2] Stucki et al Journal of Chromatography B 700 (1997) 241-248
[3] WHO Technical Report, Annex 4 Guidelines on viral inactivation and removal procedures intended to assure the viral safety of human blood plasma products Series No. 924, p 151-224, (2004).
[4] P Friberger, Scand J Clin Lab Invest Suppl, 1982;162:1-298.
[5] Remington's Pharmaceutical Sciences, 16th edition (Osol, ed. 1980).
[6] European Pharmacopoeia 6.4, chapter 2.7.13: "Assay of human anti-D immunoglobulin", 01/2008:20713

## Claims

1. A process for reducing protease activity in a solution comprising anti-D immunoglobulin G comprising the steps of:
i) providing a solution comprising anti-D immunoglobulin G;
ii) contacting said solution with aluminium hydroxide gel at a pH of 4.70 to 4.90; and
iii) separating the solution from the solid components.

2. The process of claim 1 wherein the aluminium hydroxide gel is added in an amount of 0.10-0.20g/g protein, preferably wherein the aluminium hydroxide gel is added in an amount of about 0.13g/g protein.

3. The process of claim 1 or 2 wherein step (ii) is carried out in a solution with a conductivity of 3.0 to 3.4 mS/cm, preferably wherein step (ii) is carried out in a solution which has a conductivity of about 3.2mS/cm.

4. The process of any preceding claim, wherein the mixture generated in (ii) is contacted with the aluminium hydroxide gel for a period of 15 minutes to 2 hours.

5. The process of any preceding claim, wherein the resulting solution has (a) reduced protease activity and at least the same amount of anti-D compared to the same process in which the pH in step (ii) is pH 5.5 and/or (b) reduced protease activity and an increased amount of anti-D compared to the same process in which the pH in step (ii) is pH 5.5.

6. The process of any preceding claim, wherein an anion exchange step is carried out before the step of contacting said solution with aluminium hydroxide gel, optionally wherein the anion exchange step is a DEAE step.

7. The process of any preceding claim, wherein a cation exchange step is carried out before the step of contacting said solution with aluminium hydroxide gel.

8. The process of any preceding claim, wherein a solvent detergent virus inactivation step is carried out before the step of contacting said solution with aluminium hydroxide gel, preferably wherein said solvent detergent virus inactivation step is carried out using Tri (n-butyl) phosphate (TnBP) and Polysorbate-20 (PS-20), and more preferably wherein said solvent detergent virus inactivation step is carried out using 1% TnBP (w/w) )and 1.75% PS-20 (w/w).

9. The process of any preceding claim:
(a) wherein a cation exchange chromatography step is carried out on the solution after step (iii); and/or
(b) wherein a virus filtration step is carried out on the solution after step (iii); and/or
(c) further comprising one or more of (i) formulating the solution, optionally with glycine and/or albumin, and (ii) filling syringes with the solution

10. The process of any preceding claim, wherein the solution comprising anti-D immunoglobulin G is plasma from human Rh-negative donors who have been sensitised to rhesus factor D, or a fraction thereof.

11. The process of any preceding claim, wherein the solution comprising anti-D immunoglobulin G is plasma from human Rh-negative donors who have been sensitised to rhesus factor D, or a fraction thereof, a solvent detergent virus inactivation step using 1% TnBP (w/w) )and 1.75% PS-20 (w/w) is carried out before the step of contacting said solution with aluminium hydroxide gel and wherein step (ii) is carried out by contacting said solution with aluminium hydroxide gel in an amount of 0.10-0.20g/g protein at a pH of 4.70 to 4.90 and a conductivity of 3.0 to 3.4 mS/cm.

12. The process of any preceding claim, comprising the steps of: a) providing plasma from human Rh-negative donors who have been sensitised to rhesus factor D or a fraction thereof, comprising anti-D immunoglobulin G; b) carrying out a solvent detergent virus inactivation step on the plasma or fraction thereof using Tri (n-butyl) phosphate (TnBP) and Polysorbate-20 (PS-20); c) carrying out a cation exchange chromatography step on the solvent detergent treated plasma or fraction thereof; d) carrying out an anion exchange step on the eluate from step (b); d) contacting said solution with aluminium hydroxide gel in an amount of 0.10-0.20g/g protein at a pH of 4.70 to 4.90 and a conductivity of 3.0 to 3.4 mS/cm ; and separating the solution from the solid components; e) carrying out a further step of cation exchange chromatography on the solution from d); f) filtering the solution from e) to reduce the amount of any virus present; and g) one or more of (i) formulating the solution, optionally with glycine and/or albumin, and (ii) filling syringes with the solution.

13. A process for producing an anti-D immunoglobulin G-preparation from plasma from rhesus negative blood of rhesus factor D sensibilized donors, a plasma fraction thereof, said method comprising:
i) providing a solution comprising anti-D immunoglobulin G;
ii) contacting said solution with aluminium hydroxide gel at a pH of 4.70 to 4.90; and
iii) separating the solution from the solid components.

14. The process of claim 13 wherein:
(a) the aluminium hydroxide gel is added in an amount of 0.10-0.20g/g protein, preferably wherein the aluminium hydroxide gel is added in an amount of about 0.13g/g protein; and/or
(b) step (ii) is as defined in claim 3 and/or claim 4; and/or
(c) wherein the resulting solution has:
(A) reduced protease activity and at least the same amount of anti-D; and/or
(B) reduced protease activity and an increased amount of anti-D;
compared to the same process in which the pH in step (ii) is pH 5.5; and/or
(d) wherein an anion exchange step is carried out before the step of contacting said solution with aluminium hydroxide gel, optionally wherein the anion exchange step is a DEAE step; and/or
(e) wherein a cation exchange step is carried out before the step of contacting said solution with aluminium hydroxide gel; and/or
(f) wherein a solvent detergent virus inactivation step is carried out before the step of contacting said solution with aluminium hydroxide gel, optionally wherein said solvent detergent virus inactivation step as defined in claim 8; and/or
(g)
(A) a cation exchange chromatography step is carried out on the solution after step (iii); and /or
(B) a virus filtration step is carried out on the solution after step (iii); and/or
(C) further comprising one or more of:
(i) formulating the solution, optionally with glycine and/or albumin; and
(ii) filling syringes with the solution.

15. The process of any of claims 13 to 14, wherein the solution comprising anti-D immunoglobulin G is plasma from human Rh-negative donors who have been sensitised to rhesus factor D, or a fraction thereof.

16. The process of any of claims 13 to 15, wherein the solution comprising anti-D immunoglobulin G is plasma from human Rh-negative donors who have been sensitised to rhesus factor D, or a fraction thereof, a solvent detergent virus inactivation step using 1% TnBP (w/w) )and 1.75% PS-20 (w/w) is carried out before the step of contacting said solution with aluminium hydroxide gel and wherein step (ii) is carried out by contacting said solution with aluminium hydroxide gel in an amount of 0.10-0.20g/g protein at a pH of 4.70 to 4.90 and a conductivity of 3.0 to 3.4 mS/cm.

17. The process of any of claims 13 to 16, comprising the steps of: a) providing plasma from human Rh-negative donors who have been sensitised to rhesus factor D or a fraction thereof, comprising anti-D immunoglobulin G; b) carrying out a solvent detergent virus inactivation step on the plasma or fraction thereof using Tri (n-butyl) phosphate (TnBP) and Polysorbate-20 (PS-20); c) carrying out a cation exchange chromatography step on the solvent detergent treated plasma or fraction thereof; d) carrying out an anion exchange step on the eluate from step (b); d) contacting said solution with aluminium hydroxide gel in an amount of 0.10-0.20g/g protein at a pH of 4.70 to 4.90 and a conductivity of 3.0 to 3.4 mS/cm; and separating the solution from the solid components; e) carrying out a further step of cation exchange chromatography on the solution from d); f) filtering the solution from e) to reduce the amount of any virus present; and g) one or more of (i) formulating the solution, optionally with glycine and/or albumin, and (ii) filling syringes with the solution.

## Amended claims

### Amended claims in accordance with Rule 137(2) EPC.

1. A process for reducing protease activity in a solution comprising anti-D immunoglobulin G comprising the steps of:
i) providing a solution comprising anti-D immunoglobulin G;
ii) contacting said solution with aluminium hydroxide gel at a pH of 4.70 to 4.90; and
iii) separating the solution from the solid components.

2. The process of claim 1 wherein the aluminium hydroxide gel is added in an amount of 0.10-0.20g/g protein, preferably wherein the aluminium hydroxide gel is added in an amount of about 0.13g/g protein.

3. The process of claim 1 or 2 wherein step (ii) is carried out in a solution with a conductivity of 3.0 to 3.4 mS/cm, preferably wherein step (ii) is carried out in a solution which has a conductivity of about 3.2 mS/cm.

4. The process of any preceding claim, wherein the mixture generated in (ii) is contacted with the aluminium hydroxide gel for a period of 15 minutes to 2 hours.

5. The process of any preceding claim, wherein the resulting solution has (a) reduced protease activity and at least the same amount of anti-D compared to the same process in which the pH in step (ii) is pH 5.5 and/or (b) reduced protease activity and an increased amount of anti-D compared to the same process in which the pH in step (ii) is pH 5.5.

6. The process of any preceding claim, wherein an anion exchange step is carried out before the step of contacting said solution with aluminium hydroxide gel, optionally wherein the anion exchange step is a DEAE step.

7. The process of any preceding claim, wherein a cation exchange step is carried out before the step of contacting said solution with aluminium hydroxide gel.

8. The process of any preceding claim, wherein a solvent detergent virus inactivation step is carried out before the step of contacting said solution with aluminium hydroxide gel, preferably wherein said solvent detergent virus inactivation step is carried out using Tri (n-butyl) phosphate (TnBP) and Polysorbate-20 (PS-20), and more preferably wherein said solvent detergent virus inactivation step is carried out using 1% TnBP (w/w) and 1.75% PS-20 (w/w).

9. The process of any preceding claim:
(a) wherein a cation exchange chromatography step is carried out on the solution after step (iii); and/or
(b) wherein a virus filtration step is carried out on the solution after step (iii); and/or
(c) further comprising one or more of (i) formulating the solution, optionally with glycine and/or albumin, and (ii) filling syringes with the solution

10. The process of any preceding claim, wherein the solution comprising anti-D immunoglobulin G is plasma from human Rh-negative donors who have been sensitised to rhesus factor D, or a fraction thereof.

11. The process of any preceding claim, wherein the solution comprising anti-D immunoglobulin G is plasma from human Rh-negative donors who have been sensitised to rhesus factor D, or a fraction thereof, wherein a solvent detergent virus inactivation step using 1% TnBP (w/w) and 1.75% PS-20 (w/w) is carried out before the step of contacting said solution with aluminium hydroxide gel and wherein step (ii) is carried out by contacting said solution with aluminium hydroxide gel in an amount of 0.10-0.20g/g protein at a pH of 4.70 to 4.90 and a conductivity of 3.0 to 3.4 mS/cm.

12. The process of any preceding claim, comprising the steps of: a) providing plasma from human Rh-negative donors who have been sensitised to rhesus factor D or a fraction thereof, comprising anti-D immunoglobulin G; b) carrying out a solvent detergent virus inactivation step on the plasma or fraction thereof using Tri (n-butyl) phosphate (TnBP) and Polysorbate-20 (PS-20); c) carrying out a cation exchange chromatography step on the solvent detergent treated plasma or fraction thereof; d) carrying out an anion exchange step on the eluate from step (b); d) contacting said solution with aluminium hydroxide gel in an amount of 0.10-0.20g/g protein at a pH of 4.70 to 4.90 and a conductivity of 3.0 to 3.4 mS/cm ; and separating the solution from the solid components; e) carrying out a further step of cation exchange chromatography on the solution from d); f) filtering the solution from e) to reduce the amount of any virus present; and g) one or more of (i) formulating the solution, optionally with glycine and/or albumin, and (ii) filling syringes with the solution.

13. A process for producing an anti-D immunoglobulin G-preparation from plasma from rhesus negative blood of rhesus factor D sensibilized donors, or a plasma fraction thereof, said method comprising:
i) providing a solution comprising anti-D immunoglobulin G;
ii) contacting said solution with aluminium hydroxide gel at a pH of 4.70 to 4.90; and
iii) separating the solution from the solid components.

14. The process of claim 13 wherein:
(a) the aluminium hydroxide gel is added in an amount of 0.10-0.20g/g protein, preferably wherein the aluminium hydroxide gel is added in an amount of about 0.13g/g protein; and/or
(b) step (ii) is as defined in claim 3 and/or claim 4; and/or
(c) wherein the resulting solution has:
(A) reduced protease activity and at least the same amount of anti-D; and/or
(B) reduced protease activity and an increased amount of anti-D;
compared to the same process in which the pH in step (ii) is pH 5.5; and/or
(d) wherein an anion exchange step is carried out before the step of contacting said solution with aluminium hydroxide gel, optionally wherein the anion exchange step is a DEAE step; and/or
(e) wherein a cation exchange step is carried out before the step of contacting said solution with aluminium hydroxide gel; and/or
(f) wherein a solvent detergent virus inactivation step is carried out before the step of contacting said solution with aluminium hydroxide gel, optionally wherein said solvent detergent virus inactivation step as defined in claim 8; and/or
(g)
(A) a cation exchange chromatography step is carried out on the solution after step (iii); and /or
(B) a virus filtration step is carried out on the solution after step (iii); and/or
(C) further comprising one or more of:
(i) formulating the solution, optionally with glycine and/or albumin; and
(ii) filling syringes with the solution.

15. The process of any of claims 13 to 14, wherein the solution comprising anti-D immunoglobulin G is plasma from human Rh-negative donors who have been sensitised to rhesus factor D, or a fraction thereof.

16. The process of any of claims 13 to 15, wherein the solution comprising anti-D immunoglobulin G is plasma from human Rh-negative donors who have been sensitised to rhesus factor D, or a fraction thereof, a solvent detergent virus inactivation step using 1% TnBP (w/w) and 1.75% PS-20 (w/w) is carried out before the step of contacting said solution with aluminium hydroxide gel and wherein step (ii) is carried out by contacting said solution with aluminium hydroxide gel in an amount of 0.10-0.20g/g protein at a pH of 4.70 to 4.90 and a conductivity of 3.0 to 3.4 mS/cm.

17. The process of any of claims 13 to 16, comprising the steps of: a) providing plasma from human Rh-negative donors who have been sensitised to rhesus factor D or a fraction thereof, comprising anti-D immunoglobulin G; b) carrying out a solvent detergent virus inactivation step on the plasma or fraction thereof using Tri (n-butyl) phosphate (TnBP) and Polysorbate-20 (PS-20); c) carrying out a cation exchange chromatography step on the solvent detergent treated plasma or fraction thereof; d) carrying out an anion exchange step on the eluate from step (b); d) contacting said solution with aluminium hydroxide gel in an amount of 0.10-0.20g/g protein at a pH of 4.70 to 4.90 and a conductivity of 3.0 to 3.4 mS/cm; and separating the solution from the solid components; e) carrying out a further step of cation exchange chromatography on the solution from d); f) filtering the solution from e) to reduce the amount of any virus present; and g) one or more of (i) formulating the solution, optionally with glycine and/or albumin, and (ii) filling syringes with the solution.
